# EUROPEAN PATENT SPECIFICATION

(11) **EP 2 294 966 B1**
(45) Date of publication and mention of the grant of the patent: **26.09.2012**
(21) Application number: 10015526.6
(22) Date of filing: 12.11.2004
(51) Int. Cl.: A61B 1/06

(54) **Endoscope**
Endoskop
Endoscope

(30) Priority: 12.11.2003 JP 2003382967
(43) Date of publication of application: 16.03.2011
(62) Divisional of application: 04818529.2
(73) Proprietor: Olympus Corporation, Shibuya-ku Tokyo 151-0072 (JP)
(72) Inventor: Miyagi, Masaaki, Tokyo 151-0072 (JP); Moriyama, Hiroki, Tokyo 151-0072 (JP); Takase, Seisuke, Tokyo 151-0072 (JP)
(74) Representative: Gunzelmann, Rainer

(56) References cited:
- WO-A1-03/030525
- WO-A1-03/084392
- JP-A- 9 098 943
- JP-A- 2001 258 823
- JP-A- 2002 058 633
- US-A- 4 802 460
- US-A1- 2001 035 902

## Description

### TECHNICAL FIELD

The present invention relates to an endoscope, and more particularly to an endoscope having a distinctive distal end part of an insert part.

### BACKGROUND ART

Conventionally, endoscopes are widely used in medical field and the like. The endoscope is used with an elongated insert part thereof inserted into body cavity to observe organs and the like inside body cavity as well as to perform a variety of treatments if necessary using medical instruments inserted into a medical instrument insertion channel. A bending part is provided at a distal end of the insert part, and direction of an observation window provided at a distal end part of the bending part can be shifted by manipulating a manipulator unit of the endoscope.

Angular field of view of the conventional endoscope is not more than 140°, for example and an operator observes inside the body cavity by an observation image within the angular field of view; body cavity parts lying outside of the field of view can be observed by bending the bending part. The endoscope having the angular field of view as described above is provided with two illumination windows at the distal end part of the insert part; illuminations by the two illumination windows are sufficient for the endoscope having such angular field of view.

On the other hand, an endoscope having wider angular field of view to observe wider range is proposed (for example, see Patent Document 1). This endoscope is provided with four illumination windows at a distal end part of an insert part thereof.

The four illumination windows provided at the distal end part of the insert part provide illumination over a wide field of view, thereby preventing light intensity from decreasing at a periphery of an image displayed on a monitor. However, an increase in the number of illumination windows causes an increase in the number of optical guides inserted into the insert part, and thus requires a large diameter of the insert part, in other words, a large space for the insert part.

Further, other proposed endoscope having wider angular field of view is provided with three illumination windows arranged at a distal end part of an insert part thereof (for example, see Patent Document 2).
Patent Document 1: Japanese Patent Application Laid-Open No. 2001-258823 (FIG. 3)
Patent Document 2: Japanese Patent Application Laid-Open No. H4-102432 (FIG. 3)

JP 9-098943 discloses an endoscope having an image pickup device and light guide bundles which are arranged on the outside of right, left, and lower sides of an image pickup surface of the image pickup device. The diameters of the left and right light guide bundles are almost the same and are smaller that that of the lower light guide bundles.

US 2001/035902 discloses an in vivo image acquiring unit comprising a square camera and five blue LEDs disposed equidistantly therearound.

### DISCLOSURE OF INVENTION

### PROBLEM TO BE SOLVED BY THE INVENTION

However, an issue of how to provide a good observation in the proposed endoscope, which has the three illumination windows, with well-balanced light distribution is not taken into account.

### MEANS FOR SOLVING PROBLEM

An endoscope according to an example not forming part of the present invention includes an imaging element having an imaging region of a substantially rectangular shape at an insert part thereof; an observation window provided at a distal end part of the insert part to introduce light from an object to the imaging element; and first, second, and third illumination members provided around the observation window on a distal end face of the distal end part to illuminate the object, wherein the first illumination member is arranged near a first side of the substantially rectangular shape, and each of the second and the third illumination members is arranged near two angles at both ends of a second side of the substantially rectangular shape that opposes to the first side.

An endoscope according to independent claim 1 is provided. The endoscope may include an imaging element having an imaging region of a substantially rectangular shape at an insert part thereof; an observation window provided at a distal end part of the insert part to introduce light from an object to the imaging element; and first, second, and third illumination members provided around the observation window on a distal end face of the distal end part to illuminate the object, wherein the first and the second illumination members are arranged near adjoined first and second sides of the substantially rectangular shape respectively, and the third illumination member is arranged near a second angle of the substantially rectangular shape that opposes to a first angle which is an intersection of the first and the second sides.

In the endoscope, the imaging element has an outer peripheral shape provided with two sides being substantially parallel to respective two opposing sides among sides of the substantially rectangular shape of the imaging region.

In the endoscope, an image obtained by the imaging element is processed so that the image has two sides substantially parallel to respective two opposing sides among sides of the substantially rectangular shape of the imaging region, and is displayed on a predetermined display unit.

In the endoscope according to the present invention, an image obtained by the imaging element is processed so that the image has two sides substantially parallel to respective two opposing sides of the outer peripheral shape of the imaging element, and is displayed on a predetermined display unit.

In the endoscope, the insert part includes three optical guides or at least one power supply line corresponding to the first, the second, and the third illumination members, and three built-in parts inserted therein, and distal end parts of the three built-in parts are arranged alternately with the three illumination members on the distal end face of the distal end part.

In the endoscope, one of the three built-in parts is a water pipe, and a nozzle of the distal end part of the water pipe, one of the three illumination members, and the observation window therebetween are arranged in a substantially straight line on the distal end face of the distal end part.

In the endoscope, the illumination member arranged in a substantially straight line with respect to the nozzle with the observation window therebetween is the first illumination member arranged near the first side.

In the endoscope, the observation window is arranged at a off-centered position with respect to the center of a surface of the distal end face, and a distance between one illumination member arranged on the distal end face at an opposite side from the off-centered position with respect to the center of the surface of the distal end face and a center of the observation window is greater than a distance between one of the other two illumination members and the center of the observation window, the one illumination member not being the illumination member arranged in a substantially straight line with the nozzle.

### EFFECT OF THE INVENTION

Good observation of an endoscope is provided since an observation image of an object can be taken more sharply by illuminating the object with well-balanced light.

### BRIEF DESCRIPTION OF DRAWINGS

FIG. 1 is a schematic diagram schematically showing an endoscope apparatus according to an embodiment of the present invention;
FIG. 2 is a front view seen from a distal end side of cylindrical distal end part;
FIG. 3 is a sectional view taken along line P-P of FIG. 2;
FIG. 4 is a schematic diagram illustrating a positional relationship among an observation window and three illumination windows in an example not forming part of the present invention;
FIG. 5 is a schematic diagram illustrating a positional relationship among a rectangular frame corresponding to a substantially rectangular shaped observation image and three illumination windows for
illuminating an object of the observation image in an example not forming part of the present invnetion;
FIG. 6 is a schematic diagram illustrating a positional relationship among a rectangular frame corresponding to a substantially rectangular shaped observation image and three illumination windows for illuminating an object of the observation image in an example not forming part of the present invention ;
FIG. 7 is a schematic diagram illustrating another example of a positional relationship among a rectangular frame corresponding to a substantially rectangular shaped observation image and three illumination windows for illuminating an object of the observation image;
FIG. 8 is a fragmentary cross-sectional view illustrating a structure of a distal end face that includes an illumination window provided in a direction along an opposite side of a field-of-view range in which an observation image is taken; and
FIG. 9 is a fragmentary cross-sectional view illustrating a structure of the distal end face that includes an illumination window provided in a direction along a diagonal of a field-of-view range in which an observation image is taken.

### EXPLANATIONS OF LETTERS OR NUMERALS

] : 1 endoscope
2 : manipulator unit
3 : insert part
7 : monitor
10 : distal end part
11 : imaging element
22 : observation window
23a, 23b, 23c : illumination window
41, 41a, 41b, 41c : rectangular frame

### BEST MODE(S) FOR CARRYING OUT THE INVENTION

Embodiments according to the present invention will be described below with reference to the accompanying drawings. The present invention is not limited by the embodiments below.

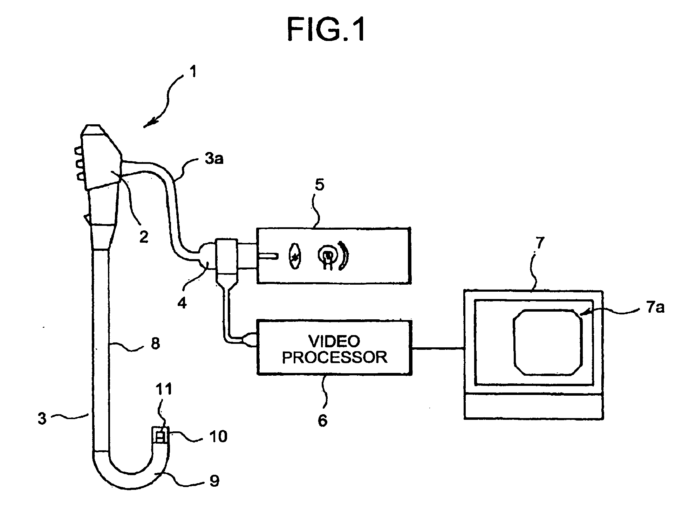

First, a configuration of an endoscope apparatus according to an embodiment will be described with reference to FIG. 1. FIG. 1 is a schematic diagram showing the endoscope apparatus according to the embodiment of the present invention. As shown in FIG. 1, the endoscope apparatus includes an endoscope 1 having a function of taking an image inside body cavity, a light source 5 emitting illumination light for taking the image to the endoscope 1, a video processor 6 that performs a predetermined imaging process on image signals sent from the endoscope 1 to form an observation image corresponding to the image signals, and a monitor 7 that displays the observation image formed by the video processor 6.

The endoscope 1 includes a manipulator unit 2 that performs bending manipulation and channel control, an insert part 3 that is inserted into body cavity and connected to the manipulator unit 2 at a proximal end thereof, and a universal cable 3a that extends from the manipulator unit 2 and has a connector 4 at a distal end thereof. The connector 4 is connected to the light source 5 and the video processor 6 through a predetermined connector. The video processor 6 is connected to the monitor 7. The insert part 3 is provided with a flexible tube 8, a bending part 9 that is provided at a distal end of the tube 8, and a distal end part 10 that is provided at a distal end of the bending part 9. The distal end part 10 has a built-in imaging element 11 that is used for taking images of parts inside body cavity.

The image signals of the parts inside the body cavity taken by the imaging element 11 provided inside the distal end part 10 are transferred to the video processor 6 through the universal cable 3a. The video processor 6 has a signal processing circuit (not shown) that processes the transferred image signals, and displays observation images of the body cavity parts based on the processed signals on a display 7a of the monitor 7, which is a display means connected to the video processor 6.

A manipulating knob that is used to remotely bend the bending part 9 is disposed in the manipulator unit 2. Manipulating the manipulating knob causes pulling and loosening of a manipulating wire (not shown) inserted into the insert part 3, thereby allowing the bending part 9 to bend in four directions.

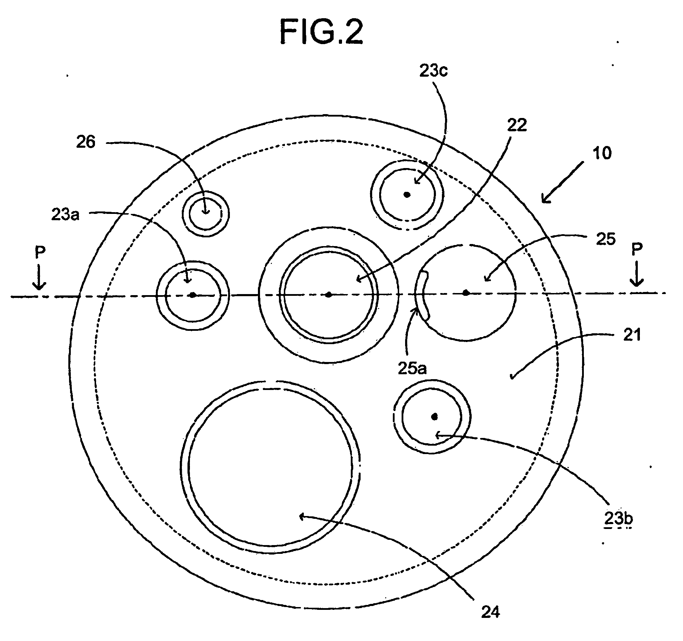

FIG. 2 is a front view seen from a distal end side of the cylindrical distal end part 10. In a distal end face 21 of the distal end part 10, an observation window 22, three illumination windows 23a, 23b, and 23c, a medical instrument opening 24, a water supply nozzle 25 that drains water, and a forward water supply nozzle 26 that washes blood, mucus, and the like of the diseased part of the subject and the like. Therefore, the distal end face 21 of the distal end part 10 is provided with a few openings for the observation window 22, the three illumination windows 23a, 23b, and 23c (hereinafter, often collectively called illumination windows 23), the medical instrument opening 24, the water supply nozzle 25, and the forward water supply nozzle 26.

As shown in FIG. 2, the three illumination windows 23 are arranged on the distal end face 21 of the distal end part 10 in such a way that the three illumination windows 23 are arranged around an optical axis of the observation window 22 at intervals of predetermined angle lay on a plane orthogonal to the optical axis. Then, the medical instrument opening 24, the water supply nozzle 25, and the forward water supply nozzle 26 are disposed around the optical axis of the observation window 22 and between the adjacent illumination windows. Specifically, the medical instrument opening 24 is disposed between the illumination windows 23a and 23b, the water supply nozzle 25 is disposed between the illumination windows 23b and 23c, and the forward water supply nozzle 26 is disposed between the illumination windows 23c and 23a. In other words, each of the three windows is disposed between two of the three illumination windows 23. A positional relationship among the three illumination windows 23 and the observation window 22 will be explained in detail later.

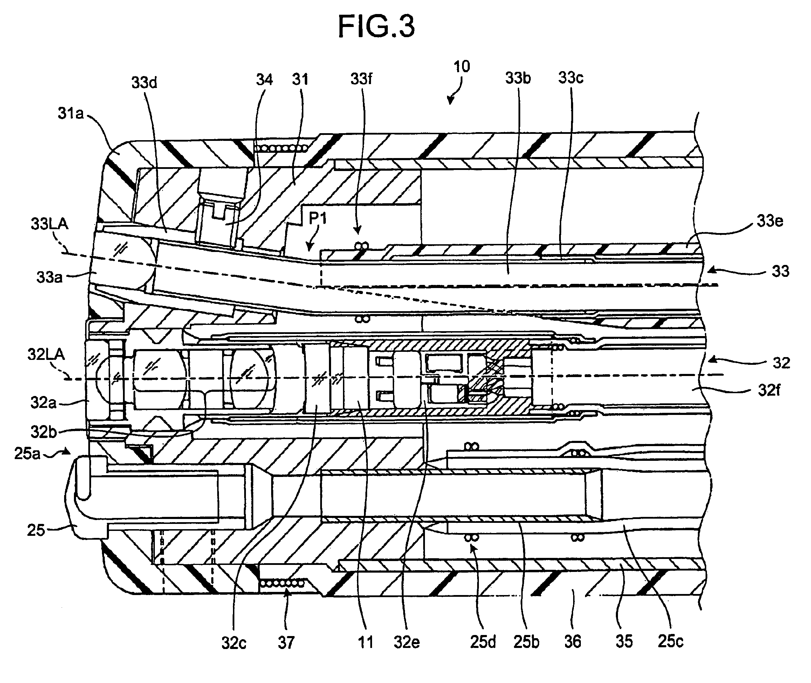

FIG. 3 is a cross-sectional view of the distal end part 10 taken along line P-P of FIG. 2. Further, a distal end rigid part 31 having a space in which an imaging unit 32 corresponding to the observation window 22 and optical guides and the like corresponding to the three illumination windows 23 can be disposed is provided in the distal end part 10. A distal end of the distal end rigid part 31 is covered by a cap 31a. The imaging unit 32 is inserted into and secured to the distal end rigid part 31 in such a way that an observation window lens 32a provided at a distal end of the imaging unit 32 is arranged at the observation window 22 of the distal end part 10. The imaging unit 32 includes the observation window lens 32a, an observation optical system 32b that is constituted of a plurality of lenses and provided at a proximal end of the observation window lens 32a, a cover glass 32c provided at a proximal end of the observation optical system 32b, and the imaging element 11, as being a solid-state imaging sensing device such as a charge-coupled device (CCD), that is provided at a proximal end of the cover glass 32c. The imaging unit 32 further includes a substrate 32e that has a variety of circuits, and the imaging element 11 is connected to the substrate 32e. Moreover, a signal cable 32f is connected to the substrate 32e. The signal cable 32f is inserted into the insert part 3, and connected to the video processor 6. The imaging unit 32 is secured to the distal end rigid part 31 by a filling material and the like not shown. The imaging element 11 has a substantially rectangular shaped (substantially quadrangular shape in the present embodiment) imaging region (referred to as image area or effective imaging region), and the imaging element 11 is formed into a substantially quadrangular shape having at least two sides being substantially parallel to each of opposing two sides of the substantial quadrangle of the imaging region (for example, a substantial quadrangle having four sides being parallel to respective sides of the substantial quadrangle of the imaging region). In the present embodiment, the imaging region has a quadrangular shape. Rectangular shape or square shape can be used as the quadrangle.

An optical guide unit 33 includes an illumination lens 33a and an optical fiber bundle 33b which is the optical guide. A distal end part of the optical fiber bundle 33b is secured inside a metal pipe 33c by bonding agent and the like. The distal end part of the optical fiber bundle 33b and the illumination lens 33a are inserted into and secured inside a frame 33d. The optical guide unit 33 is secured to the distal end rigid part 31 by a securing screw 34. A portion of the metal pipe 33c and the optical fiber bundle 33b are covered by an outer cover tube 33e. The outer cover tube 33e is secured to the metal pipe 33c by a reel 33f. The metal pipe 33c is bent in the middle at a predetermined position P1, so that the optical fiber bundle 33b is bent along the bent shape of the metal pipe 33c as shown in FIG. 3. Therefore, an optical axis 33LA of the illumination lens 33a that emits illumination light is not parallel to an optical axis 32LA of the imaging unit 32. Particularly, the optical axis 33LA is inclined with respect to the optical axis 32LA in a direction that an extending direction of the optical axis 33LA is away from the observation direction of the optical axis 32LA of the imaging unit 32. Each optical axe of the optical guide units corresponding to other illumination windows 23b and 23c is also inclined with respect to the optical axis 32LA in the direction that an extending direction of the optical axis of the optical guide unit is away from the observation direction of the optical axis 32LA of the imaging unit 32.

An opening 25a is provided at a distal end part of the water supply nozzle 25. The opening 25a is provided in such a way that water from the water nozzle 25 spurt in the direction approximately parallel to a plane that is orthogonal to the optical axis 32LA of the imaging unit 32, as well as in the direction that water spurt towards the surface of the observation window lens 32a provided at the observation window 22 and towards the surface of the illumination lens 33a provided at the illumination window 23a. A proximal end of the pipe-like water supply nozzle 25 is connected to a water supply tube 25c through a connecting pipe 25b. Hence, a water supply channel is formed by the connecting pipe 25b and the water supply tube 25c. The water supply tube 25c is secured to the connecting pipe 25b by a reel 25d.

A proximal end part of the distal end rigid part 31 is secured to a portion of a bending distal end frame 35. The proximal end of the distal end rigid part 31 and the bending distal end frame 35 are covered by the outer cover tube 36. The outer cover tube 36 is secured to the distal end rigid part 31 by a reel 37.

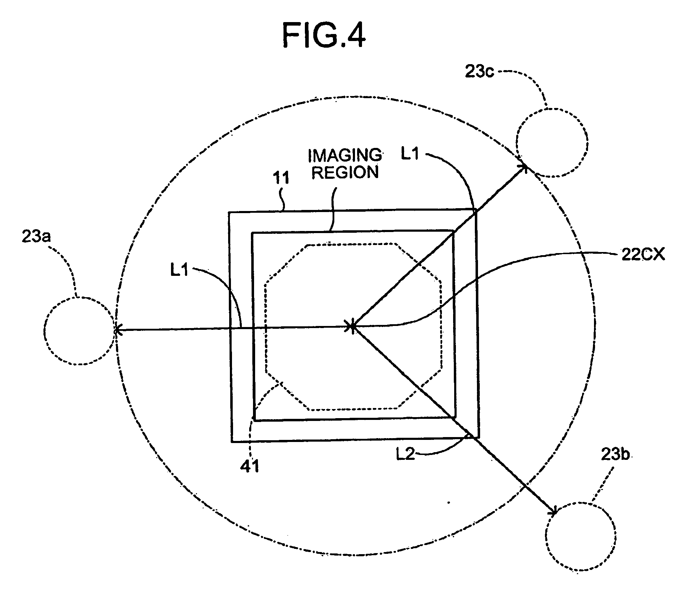

Next, a positional relationship among the observation window 22 and the three illumination windows 23 at the distal end part 10 will be explained in detail. As shown in FIG. 2, shape of a cross section of the distal end part 10 lying on a plane orthogonal to the optical axis 32LA of the observation optical system is circular. The observation window 22 is arranged on the distal end face 21 of the distal end part 10 in such a way that the center of the observation window 22 is placed off-center with respect to the center of the circular cross section described above. In the present embodiment, the center 22CX of the observation window 22 matches the optical axis 32LA of the observation optical system, and the imaging element 11 is arranged in such a way that the center of the imaging region of the imaging element 11 substantially matches the center 22CX of the observation window 22 (or the optical axis 32LA of the observation optical system) at a cross section lying on a plane orthogonal to the optical axis 32LA of the observation optical system (FIG. 4). Each of the three illumination windows 23 is arranged on the distal end face 21 of the distal end part 10 with a predetermined distance around the observation window 22. FIG. 4 is a schematic diagram illustrating the positional relationship among the observation window 22 and the three illumination windows 23 in an example not forming part of the present invention. As shown in FIG. 4, the distance from the center 22CX of the observation window 22 to each of the illumination windows 23a and 23c is a distance of L1, and to the illumination window 23b is a distance of L2. The distance L1 is shorter than the distance L2. In other words, of the two illumination windows 23b and 23c, except for the illumination window 23a in which the illumination window 23a, the water supply nozzle 25, and the observation window 22 therebetween are arranged in a substantially straight line, the illumination window 23b arranged on the distal end face 21, arranged on a side opposite to the off-centered side of the observation window 22, is placed away from the center 22CX by a distance greater than the distance between each of the other illumination windows 23a and 23c and the center 22CX. In the present embodiment, the imaging element 11 is formed in such a way that outer periphery shape (external shape) of the imaging element 11 has a rectangular shape having two substantially parallel sides with respect to each of the opposing two sides among the sides of the rectangular shaped imaging region (for example, a rectangular shape similar to the substantially rectangular shaped imaging region or a rectangular shape having four sides being parallel to respective sides of the substantially rectangular shaped imaging region). In the present embodiment, as one of the example of the shape of the imaging element 11 described above, the imaging element 11 is formed in substantially quadrangular shape having each side being parallel to each side of the imaging region, as shown in FIG. 4. Here, the external shape of the imaging element referred to an exterior shape of a semiconductor element substrate (or a semiconductor chip) provided with the imaging region.

The reason for providing one of the illumination windows with the distance L2 greater than the distance L1 of the other illumination windows is given below. Having two illumination windows is sufficient when the endoscope is provided with narrow angular field of view; however, having more than three illumination windows is required when the endoscope is provided with the wide angular field of view as described above since it is required to illuminate the wide range. However, when more than three illumination windows are to be provided, one additional optical guide needs to be incorporated into the narrow distal end part. Then a step of assembling the distal end part with various built-in parts becomes more complicated.

To alleviate this inconvenience, one of the illumination windows 23, for example 23b, is provided farther away from the observation window 22 than the other illumination windows 23. Consequently, it becomes easier to build in the optical guide unit 33 corresponding to the illumination window 23 at last while building in the built-in parts such as the optical guide unit for the illumination windows 23 into the distal end part 10. Specifically, when the optical guide unit corresponding to the illumination window 23b is inserted into the narrow space after channels used for the imaging unit 32, each of the optical guide units 33 corresponding to the two illumination windows 23a and 23c, the water supply nozzle 25, and the forward water supply nozzle 26 are inserted closely-spaced with each other, it becomes easier to insert the optical guide unit for the illumination window 23c, whereby the distal end part 10 can be fabricated more efficiently. This is because the distance L2 between the observation window 22 and the illumination window 23b is greater than the distance L1 between the observation window 22 and each of the illumination windows 23a and 23c.

Further, other than the imaging unit 32, three optical guides that are the optical fiber bundles corresponding to the respective three illumination windows 23 and medical instrument channels and the like that are the three built-in parts corresponding respectively to the medical instrument opening 24, the water supply nozzle 25, and the forward water supply nozzle 26, are inserted into the insert part 3. It is required not to increase a diameter of the insert part 3 since the six built-in parts are provided in the distal end part 10 in addition to the imaging unit 32, as described above. Thus, as shown in FIG. 2, the endoscope having the wide angular field of view can emit well-balanced illumination light, and the endoscope can prevent the diameter of the insert part 3 to be increased since each of the medical instrument opening 24, the water supply nozzle 25, and the forward water supply nozzle 26, that are distal ends of the three built-in parts, is disposed between the two of the three illumination windows 23 alternately.

Further, as shown in FIG. 2, the water supply nozzle 25 provided at a distal end part of the water supply channel and the illumination window 23a are arranged in a substantially straight line represented by P-P on the distal end face 21 of the distal end part 10 of the insert part 3, and the observation window 22 is arranged between the water supply nozzle 25 and the illumination window 23a. This is because, even if dirt adheres on the distal end face 21 of the distal end part 10 of the insert part 3, the dirt can be removed from the observation window 22 and at least from the illumination window 23a of the illumination windows 23 by water from the opening 25a of the water supply nozzle 25. Consequently, a good observation is provided since the endoscope can prevent the observation image from turning to be pitch-black. Here, the blockage of the illumination light emitted toward the object is caused by the adhered dirt and the like onto the distal end face 21 of the distal end part 10. Particularly, a center of the water supply nozzle 25 and a center of the illumination window 23a are at point symmetric position with respect to the center of the observation window 22, in FIG. 2.

Next, a relationship between the shape of the imaging region of the imaging element 11 and a display shape of the observation image displayed on the monitor 7 will be described. The imaging element 11 transfers the image signals to the video processor 6 by the incident light emitted through the observation window 22. The video processor 6 also performs electric masking process (corresponding to one of the example of the imaging process according to claims) in order to display the substantially rectangular shaped observation image corresponding to the image signals, while performing the imaging process to the received image signals. By performing the electric masking process, the observation image is displayed on the monitor 7 in a shape corresponding to the substantially rectangular shape, which is the external shape of the imaging element 11, or a shape corresponding to the substantially rectangular shaped imaging region of the imaging element. Such shapes are represented, for example, by a shape having two sides substantially parallel to each of the opposing two sides among the sides of the substantially rectangular shaped imaging element 11, or a shape having two sides substantially parallel to each of the two opposing two sides among the sides of the substantially rectangular shaped imaging region of the imaging element 11. Specifically, the observation image to be displayed on the monitor 7 as shown in FIG. 1 is displayed on the monitor 7 as an octagonal shaped image having few sides with the four corners of the substantially rectangular shaped imaging region chopped, as a result of the electric masking. Here, the few sides include at least two sides being parallel to each of the opposing two sides of the substantially quadrangular shaped imaging region (for example, four sides that are parallel to respective sides of the substantially rectangular shaped imaging region). In other words, an octagonal rectangular frame 41 showing the display shape of the observation image displayed on the monitor 7 is formed by the electric masking process on a surface perpendicular to the optical axis 32LA as shown in FIG. 4, and interior region of the rectangular frame 41 is displayed on the monitor 7 as the observation image. In the rectangular frame 41, at least opposing two sides correspond to the opposing two sides of the imaging region of the imaging element 11, and the three illumination windows 23 are arranged around the imaging region (furthermore, the rectangular frame 41) of the imaging element 11, as shown in FIG. 4. Here, "the rectangular frame" may be a substantially rectangular shape which actually is octagonal, for example.

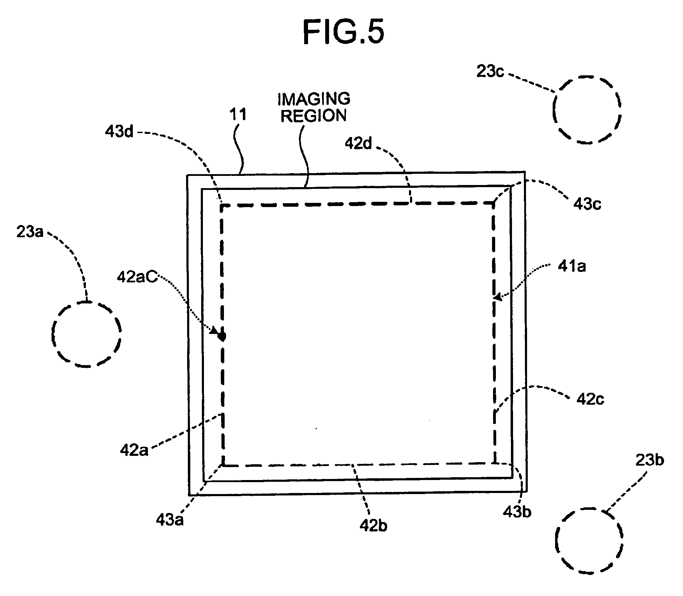

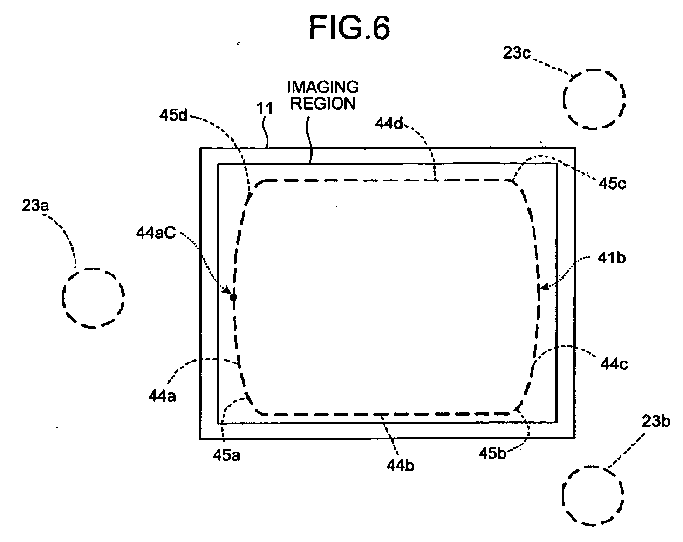

Further, a positional relationship of the three illumination windows 23 with respect to the imaging region of the imaging element 11 and the rectangular frame 41 is shown in FIGS. 5 and 6, in an example not forming part of the present invention. FIGS. 5 and 6 are schematic diagrams illustrating the positional relationship of the three illumination windows that illuminate the object of the observation image, with respect to the imaging element, the imaging region of the imaging element, and the rectangular frame that is the shape of the electric mask. Although the distance from the observation window 22 to one of the illumination windows is greater than the distance from the observation window 22 to each of the other illumination windows in FIG. 4, the three illumination windows 23 in FIGS. 5 and 6 are shown as if each of the illumination windows 23 is arranged in equal distance from the observation window 22, in order to simplify the explanation. Further, the octagonal rectangular shape is approximately shown as quadrangle, in FIG. 5.

A rectangular frame 41a of FIG. 5 has four sides and four angles. Specifically, the four sides are side 42a, 42b, 42c, and 42d of the rectangular frame 41a, and the four angles are angle 43a, 43b, 43c, and 43d located at four corners of the rectangular frame 41a, in FIG. 5. As described above, the imaging element 11 and the imaging region of the imaging element 11 have substantially quadrangular shape. The sides and the angles of the imaging element 11 and the substantially quadrangular shaped imaging region of the imaging element 11 are arranged in such a way that the sides and the angles correspond to four sides and four angles of the approximate rectangular frame 41a respectively (or arranged in a way such that the opposing two sides (for example, side 42b and 42d) of the rectangular frame 41a correspond to the opposing two sides of the imaging element 11 and the imaging region of the imaging element 11 (two sides parallel to each other in upward and downward direction, in the drawing)). In this case, the imaging element 11, the imaging region of the imaging element 11, and the rectangular frame 41a are, for example, substantially similar to each other in shape, and each side arranged accordingly is parallel to each other. Therefore, a positional relationship of the illumination windows is explained with respect to the approximate rectangular frame 41a below, in order to simplify the explanation.

The illumination window 23a is arranged at the distal end face 21 of the distal end part 10 in such a way that the illumination window 23a is arranged near side (near the side 42a) with respect to one of the side 42a of the rectangular frame 41a. In the description, an illumination window to be located near side of a rectangle frame implies that a distance from the illumination window to the mid point of the side is shorter than a distance from the illumination window to each of angles at the both ends of the side. In FIGS. 5 to 7, the illumination windows arranged near side are placed at position nearest to the mid point of the sides 42aC, 44aC, 46aC, and 46bC.

Therefore, in the rectangular frame 41a shown in FIG. 5, the light emitted from the illumination window 23a is emitted toward the center of the rectangular frame 41a located on the right of the mid point 42aC laying on the left side 42a of the rectangular image 41a. In other words, the emitted light from the illumination window 23a illuminates a field-of-view range corresponding to a range lying from the center of the rectangular frame 41a to the side 42a. In still other words, the emitted light from the illumination window 23a illuminates a range lying from the center of the imaging region to the left side of FIG. 5 that corresponds to the side 42a of the rectangular frame 41a.

Further, the other illumination windows 23b and 23c are arranged on the distal end face 21 of the distal end part 10 in such a way that each of the two illumination windows 23b and 23c is arranged near the angle (near the angle 43b, near the angle 43c) with respect to the angles 43b and 43c at the ends of the side 42c that opposes to the side 42a of the rectangular frame 41a. In the description, having an illumination window near angle of a rectangular frame implies that a distance between the illumination window and the angle is shorter than a distance between the illumination window and each of mid points lying on both sides of the angle. In FIG. 5 to FIG. 7, the illumination windows located near the angles are arranged at positions closest to the angle 43b, 43c, 45b, 45c, and 47c.

Therefore, in the rectangular frame 41a shown in FIG. 5, the emitted light from the illumination windows 23b and 23c illuminate towards the center of the rectangular frame 41a from the two angles 43b and 43c located on the right side of the rectangular frame 41a. In other words, the lights emitted from the illumination windows 23b and 23c illuminate a range lying from the center of the imaging region to lower and upper right angles of FIG. 5 that correspond to the angles 43b and 43c of the rectangular frame 41a, respectively

Although the rectangular frame 41a, which is the shape of the electric mask, has the rectangular shape approximately similar to quadrangle in FIG. 5, a substantially rectangular shape described as recited in the description includes a shape in which a pair of sides opposing to each other is straight and other pair of sides opposing to each other is bent. In this case, the rectangular frame 41b has a shape shown in FIG. 6. Specifically, the four sides are side 44a, 44b, 44c, and 44d of the rectangular frame 41b, and the four angles are angle 45a, 45b, 45c, and 45d of the rectangular frame 41b, in FIG. 6. The shape (exterior shape) of the imaging element 11 is formed in a shape corresponding to the shape of the imaging region (the shape having two sides parallel to each of the opposing two sides of the imaging region) by the arrangement of rectangular electric mask so that the two straight lines (two sides) of the imaging region and the two straight lines (two sides 44b and 44d) of the rectangular frame 41b be parallel, respectively.

The illumination window 23a is arranged on the distal end face 21 of the distal end part 10 in such a way that the illumination window 23a is arranged near side (near side 44a) with respect to one of the side 44a of the rectangular frame 41b. Further, the other illumination windows 23b and 23c are arranged on the distal end face 21 of the distal end part 10 in such a way that each of the illumination windows 23b and 23c are arranged near angles (near the angle 45b, near the angle 45c) with respect to the angles 45b and 45c of the both ends of the side 44c that opposes to the side 44a of the rectangular frame 41b.

By arranging the three illumination windows 23 near two angles and near one side of the rectangular frame 41b, the illumination light emitted from the three illumination window 23 illuminate the field-of-view range corresponding to overall range enclosed by the rectangular frame 41b (furthermore, effective imaging region), similar to the case in which the three illumination windows 23 are arranged near side and near angle of the rectangular frame 41a described above.

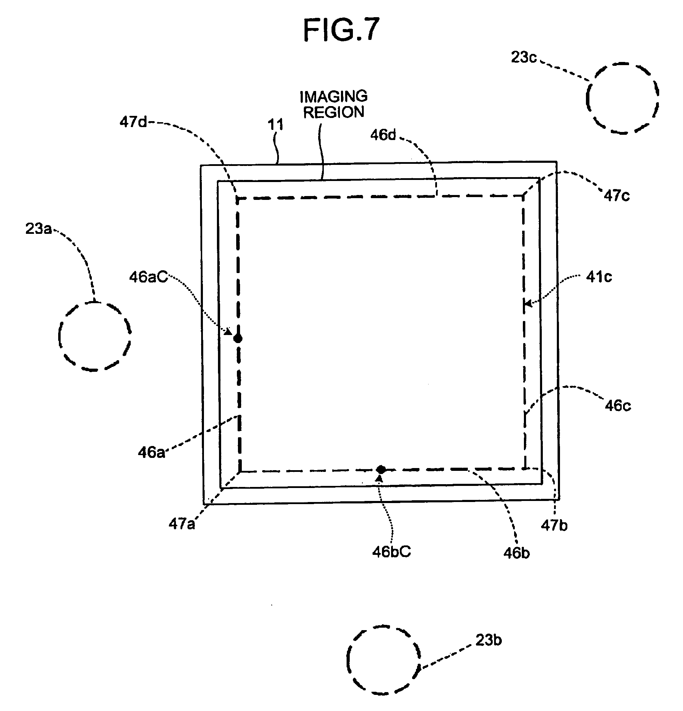

Further, another example of a positional relationship among the rectangular frame and the illumination windows 23 will be explained below. FIG. 7 is a schematic diagram illustrating another example of the positional relationship among the rectangular frame and the three illumination windows that illuminate the object of the image. Here, the three illumination windows 23 are shown in such a way that each of the three illumination windows 23 are arranged in equal distance from the observation window 22, and the octagonal rectangular frame is approximated to quadrangular shape in order to simplify the explanation, similar to those in FIG. 5 and FIG. 6. Furthermore, the imaging element 11 and the imaging region of the imaging element 11 have substantially quadrangular shape, and the sides and the angles of the imaging element 11 and the imaging region of the imaging element 11 correspond to the four sides and four angles of the approximated rectangular frame, respectively.

The rectangular frame 41c shown in FIG. 7 includes four sides and four angles. Specifically, the four sides of the rectangular frame 41c are sides 46a, 46b, 46c, and 46d, and the four angles of the rectangular frame 41c are angles 47a, 47b, 47c, and 47d, in FIG. 7.

The two illumination windows 23a and 23b are arranged on the distal end face 21 of the distal end part 10 in such a way that each of the two illumination windows 23a and 23b are arranged near each of the sides (near the side 46a and near the side 46b) with respect to adjoining two sides 46a and 46b of the rectangular frame 41c.

Therefore, light emitted from the two illumination windows 23a and 23b travels towards the two sides 46c and 46d that oppose to the sides 46a and 46b of the rectangular frame 41c, in the rectangular frame 41c of the observation window 22. In other words, the emitted light from the two illumination windows 23a and 23b illuminate a visual field range corresponding to a range lying among the center of the rectangular frame 41c, the side 46a, and the side 46b. In still other words, the emitted light from the two illumination windows 23a and 23b illuminates left and lower side imaging region of FIG. 7 in which each region corresponds to the region lying between the center of the imaging region and the side 46a of the rectangular frame 41c, and the region lying between the center of the imaging region and the side 46b of the rectangular frame 41c.

Further, the illumination window 23c is arranged on the distal end face 21 of the distal end part 10 in such a way that the illumination window 23c is arranged near angle (near the angle 47c) with respect to the angle 47c opposing to the angle 47a, which is an intersection of the adjoining sides 46a and 46b of the rectangular frame 41c.

Therefore, the emitted light from the illumination window 23c travels from the opposing angle 47c that opposes to one of the angle 47a which is an intersection of the adjoining two sides 46a and 46b of the rectangular frame 41c, to the angle 47a that is diagonal to the angle 47c. In other words, the light emitted from the illumination window 23c illuminates a visual field range corresponding to a range lying from the center of the rectangular frame 41c to the angle 47c. In still other words, the light emitted from the illumination window 23c illuminates upper right region of the effective imaging region of FIG. 7 that corresponds to the region lying between the center of the imaging region and the angle 47c of the rectangular frame 41c.

By arranging the three illumination windows 23 near two sides and one angle of the rectangular frame 41c, the illumination light emitted from the three illumination windows 23 illuminate the field-of-view range corresponding to overall range enclosed by the rectangular frame 41c (furthermore, effective imaging region).

Here, diameter of the optical guide connected to the illumination window 23a arranged near side in FIGS. 5 and 6 and diameter of the optical guides connected to each of the illumination windows 23a and 23b arranged near sides in FIG. 7 can be greater than the diameter of the optical guides connected to other illumination windows located near angles, which are the illumination windows 23b and 23c in FIGS. 5 and 6, and illumination window 23c in FIG. 7. This is because each of the illumination windows arranged near side is required to illuminate two vicinity angles. The two angles are the angles 43d and 43a in FIG. 5, the angles 45d and 45a in FIG. 6, and angles 47d and 47a, and angle 47a and 47d in FIG. 7. Increasing the diameter of the optical guide implies increasing the number of the fiber optics.

The two angles can be illuminated more brightly by increasing the number of optical fibers of the optical guide connected to the illumination windows arranged near side, since the light intensity illuminating the field-of-view range corresponding to the two angles increases.

Further, although the example in which the illumination window arranged on the substantially straight line with the water supply nozzle while locating the observation window 22 between the illumination window and the water supply nozzle 25 is the illumination window located near side as shown in the embodiment of the present invention, the present invention is not limited to the example. Thus, the illumination window arranged on the substantially straight line with the water supply nozzle while locating the observation window 22 between the illumination window and the water supply nozzle 25 can be replaced by the illumination window located near angle. Further, when there are two illumination windows located near sides as shown in FIG. 7, the illumination window arranged on the substantially straight line with the water supply nozzle while locating the observation window 22 between the illumination window and the water supply nozzle 25 can be replaced by any of the illumination windows located near two sides.

The illumination light emitted from the three illumination windows can illuminate the object in the imaging region most effectively by well balancing distribution of the light emitted from the three illumination windows, in the endoscope having wide angular field of view. The well-balanced light distribution is obtained by arranging the three illumination windows with respect to the rectangular frame corresponding to the electric mask shape of the generated image and the shape of the imaging region, as described in the positional relationship above. Further, inside of the imaging region can be illuminated effectively by forming the shape of the imaging element as a shape corresponding to the imaging region, which is a shape provided with at least two sides parallel to each of the opposing two sides of the imaging region (for example, substantially quadrangular shape provided with four sides parallel to respective sides of the imaging region), while preventing the insert part distal end part from becoming thick as much as possible.

The surface of the illumination window 23 arranged on the distal end part 10 of the insert part 3 is not arranged in parallel with respect to the plane orthogonal to the optical axis 32LA of the imaging unit 32, as shown in FIG. 3. This is because each of the optical axes, for example the optical axis 33LA, of the illumination window 23 is inclined with respect to the optical axis 32LA of the observation window 22; therefore the surface of the illumination windows 23 and the surface of the observation window 22 are not in parallel.

Further, the diagonal angle of view is wider than the vertical or horizontal angle of view in the range of the field of view from which the substantially rectangular observation image is taken. Thus, an angle (this will be referred to diagonal illumination window inclination angle, hereinafter) between the diagonal direction of the field-of-view range of the generated observation image and the surface of the observation window 22 is set to be greater than an angle (this will be referred to opposite side illumination window inclination angle, hereinafter) between the opposite side direction of the field-of-view range of the generated observation image and the surface of the observation window 22. In other words, the angle between a surface including a surface of the illumination windows 23 provided near angle in the rectangular frame 41a, 41b, and 41c described above, and the surface of the observation window is set to be greater than a surface including a surface of the illumination windows 23 provided near side in the rectangular frame 41a, 41b, and 41c described above, and the surface of the observation window 22. Consequently, flare is difficult to be caused on the observation image since light from the illumination windows 23 located at the diagonal direction, which is a direction having wider angle of view, is difficult to be incident on the observation window 22.

Detailed explanation of above description will be given in the following with reference to the drawings.

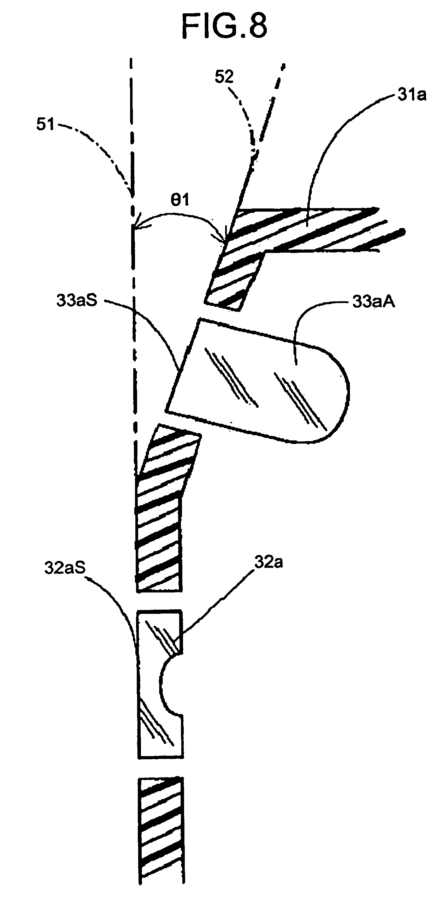

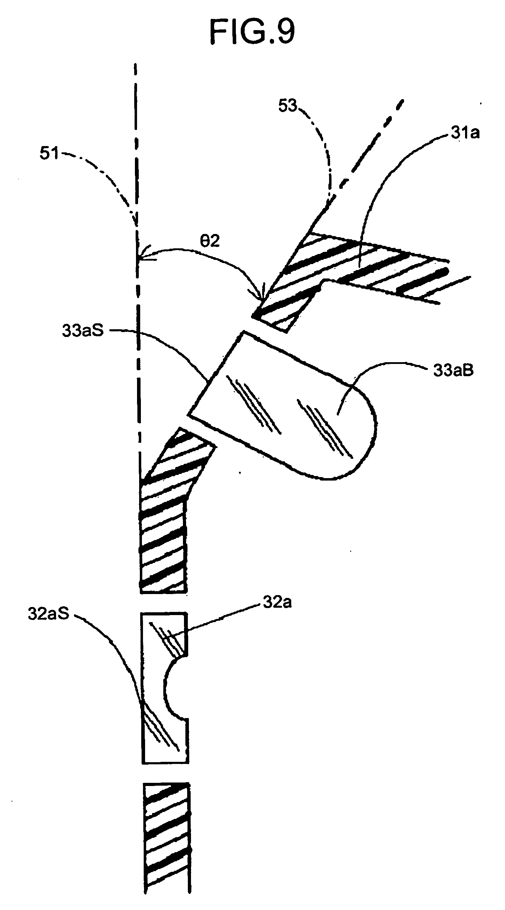

FIGS. 8 and 9 are fragmentary cross-sectional views illustrating an arrangement of a portion of the distal end face 21 of the distal end part 10 of the insert part 3. Here, FIGS. 8 and 9 only show components necessary for the explanation, in order to simplify the explanation.

FIG. 8 is a fragmentary cross-sectional view illustrating a structure of the distal end face 21 that includes the illumination windows 23 provided in a direction along the opposite side of the field-of-view range in which the generated observation image is taken. FIG. 9 is a fragmentary cross-sectional view illustrating a structure of the distal end face 21 that includes the illumination windows 23 provided in a direction along the diagonal of the field-of-view range in which the generated observation image is taken.

In FIG. 8, 33aA is an illumination lens that is arranged at the illumination window 23 provided in the direction along the opposite side of the field-of-view range in which the generated observation image is taken. Therefore, the opposite side illumination window inclination angle between a surface 51 including a surface 32aS of the observation lens 32a and a surface 52 including a surface 33aS of an illuminating lens 33aA is indicated by θ1 at the distal end face of the distal end part 10 of the insert part 3 as shown in FIG. 8.

In FIG. 9, 33aB is an illumination lens arranged at the illumination window 23 provided in a direction along the diagonal of the field-of-view range in which the generated observation image is taken. Therefore, a diagonal illumination window inclination angle between the surface 51 including a surface 32aS of the observation lens 32a and a surface 53 including the surface 33aS of the illumination lens 33aB at the distal end face of the distal end part 10 of the insert part 3 is indicated by θ2 as shown in FIG. 9.

Then, the distal end face of the distal end part 10 is formed in such a way that the diagonal illumination window inclination angle θ2 is set to be greater than the opposite side illumination window inclination angle θ1 in order to make it difficult for light from the illumination windows 23 located near angle in the direction with a wider angle of view to be incident onto the observation window 22.

The illumination windows 23 located in the direction with a wider angle of view are the illumination windows 23b and 23c in FIGS. 4 and 6 described above, and illumination window 23c in FIG. 7 described above, in which the illumination windows are located near the angles. The illumination windows located near side are the illumination window 23a in FIGS. 4 and 6 described above, and the illumination windows 23a and 23b in FIG. 7 described above. Particularly, since inclination angle of the surface of the illumination window 23a located in the direction to be exposed to water from the opening 25a of the water supply nozzle 25 is smaller than the inclination angles of other illumination windows 23b and 23c, the surface of the illumination window 23a is washed easily by water compared to the other illumination windows 23b and 23c.

Although the illumination windows 23a, 23b, and 23c, and the optical guides corresponding to each of the illumination windows are provided in the embodiment given above, the present invention is not limited to the above embodiment, and illuminating means such as an LED and the like can be provided at the locations of the illumination windows 23a, 23b, and 23c, and electric power supply line that supplies electric power to the illuminating means can be provided in the insert part 3. The electric power supply line includes a signal line that controls the illumination of the illuminating means. In other words, emission of the illumination light from the illumination windows 23a, 23b, and 23c is required only, and the illumination members including the illumination windows 23a, 23b, and 23c and the illuminating means is arranged on the position of the illumination windows 23a, 23b, and 23c, described above.

Further, the rectangular frame described above can have a shape of the imaging element 11 itself, or a shape of the imaging region. In other words, although the imaging element 11 and the imaging region have quadrangular shape in the present disclosure, the present disclosure is not limited to the above embodiment, and the imaging element 11 and the imaging region can have the substantially octagonal shape shown in FIG. 4 or the shape shown in FIG. 6. When the imaging element 11 and the imaging region have the shapes shown in FIGS. 4 or 6, an intersection of adjoining two sides is referred to as an angle, similar to those in the rectangular frame. When the shape of the imaging element 11 or the shape of the effective imaging region is the shape shown in FIG. 6, the angle described above corresponds to the angle 45a, 45b, 45c, and 45d.

According to the present embodiment as described above, the endoscope having three illumination windows can realize the endoscope with a good observation by well-balanced light distribution of the three illumination windows.

### INDUSTRIAL APPLICABILITY

The endoscope according to the present invention is useful for a process in which an image of an object is taken sharply while effectively illuminating inside of body cavity of the object, and particularly suitable for an endoscope apparatus used for observing inside of the body cavity of the object while displaying the image of inside body cavity of the object.

## Claims

1. An endoscope (1) which includes an imaging element (11) having an imaging region at an insert part (3) thereof, comprising:
an observation window (22) provided at a distal end part (10) of the insert part (3) to introduce light from an object to the imaging element (11); and
first, second, and third illumination members (23a-c) provided around the observation window (22) on a distal end face of the distal end part (10) to illuminate the object,
wherein:
sides and angles of the imaging element (11) and the imaging region of the imaging element (11) correspond to four sides and four angles of a substantially rectangular frame (41);
the first and the second illumination members (23a-b) are arranged near adjoined first and second sides (46a-b) of the substantially rectangular frame (41) respectively such that:
a distance from the first illumination member (23a) to a midpoint of the first side (46a) is shorter than a distance from the first illumination member (23a) to each of two angles (47a, 47d) of the rectangular frame (41) located at both ends of the first side (46a), and
a distance from the second illumination member (23b) to a midpoint of the second side (46b) is shorter than a distance from the second illumination member (23b) to each of two angles (47a, 47b) of the rectangular frame (41) located at both ends of the second side (46b);
the third illumination member (23c) is arranged near a second angle (47c) of the substantially rectangular frame (41) that opposes to a first angle (47a) which is an intersection of the first and the second sides (46a-b) such that a distance between the third illumination member (23c) and the second angle (47c) is shorter than a distance between the third illumination member (23c) and respective midpoints of sides (46c, 46d) lying on both sides of the second angle (47c);
the insert part (3) includes three optical guides (33) corresponding to the first, the second, and the third illumination members (23a-c) or at least one electric power supply line to supply electric power to the first, second and third illumination members, and three built-in parts (24-26) inserted therein;
distal end parts of the three built-in parts are arranged alternately with the three illumination members (23a-c) on the distal end face of the distal end part (10);
one of the three built-in parts is a water pipe (25b);
a nozzle (25) of the distal end part of the water pipe (25b), the first illumination member (23a) arranged near the first side (46a), and the observation window (22) therebetween are arranged in a substantially straight line (P-P) on the distal end face of the distal end part (10); and
the observation window (22) is arranged at an off-centered position with respect to the center of a surface of the distal end face (10);
**characterized in that** a distance (L2) between one illumination member (23b) arranged on the distal end face (10) at an opposite side from the off-centered position with respect to the center of the surface of the distal end face (10) and a center of the observation window (22) is greater than a distance (L2) between one of the other two illumination members (23b) and the center of the observation window (22), the one illumination member (23a-c) not being the illumination member (23a) arranged in a substantially straight line with the nozzle (25).

2. The endoscope according to claim 1, wherein the imaging element (11) has an outer peripheral shape provided with two sides being substantially parallel to respective two opposing sides among sides of the substantially rectangular frame (41) of the imaging region.

3. The endoscope according to claim 1, further comprising a video processor (6) and a monitor (7), wherein the processor is configured to process an image obtained by the imaging element (11) so that the image has two sides substantially parallel to respective two opposing sides among sides of the substantially rectangular frame (41) of the imaging region, and monitor display it on a predetermined monitor (7).

4. The endoscope according to claim 2, further comprising a video processor (6) and a monitor (7), wherein the processor is configured to process an image obtained by the imaging element (11) so that the image has two sides substantially parallel to respective two opposing sides of the outer peripheral frame of the imaging element (11), and monitor display it on a predetermined monitor (7).

## Patentansprüche

1. Endoskop (1), das an seinem Einführteil (3) ein Bildgebungselement (11) mit einem Bildgebungsbereich enthält, mit
einem Beobachtungsfenster (22), das an einem distalen Endteil (10) des Einführteils (3) vorgesehen ist, um Licht von einem Objekt zum Bildgebungselement (11) zu leiten; und
ersten, zweiten und dritten Beleuchtungselementen (23a-c), die an einer distalen Endfläche des distalen Endteils (10) um das Beobachtungsfenster (22) herum vorgesehen sind, um das Objekt zu beleuchten,
wobei
Seiten und Winkel des Bildgebungselements (11) und der Bildgebungsbereich des Bildgebungselements (11) vier Seiten und vier Winkeln eines im Wesentlichen rechtwinkligen Rahmens (41) entsprechen;
das erste und das zweite Beleuchtungselement (23a-b) in der Nähe von jeweils angrenzenden ersten und zweiten Seiten (46a-b) des im Wesentlichen rechtwinkligen Rahmens (41) so angeordnet sind, dass
ein Abstand vom ersten Beleuchtungselement (23a) zu einem Mittelpunkt der ersten Seite (46a) kürzer ist als ein Abstand vom ersten Beleuchtungselement (23a) zu jedem der beiden Winkel (47a, 47d) des rechtwinkligen Rahmens (41), die an beiden Enden der ersten Seite (46a) liegen, und
ein Abstand vom zweiten Beleuchtungselement (23b) zu einem Mittelpunkt der zweiten Seite (46b) kürzer ist als ein Abstand vom zweiten Beleuchtungselement (23b) zu jedem der beiden Winkel (47a, 47b) des rechtwinkligen Rahmens (41), die an beiden Enden der zweiten Seite (46b) liegen;
das dritte Beleuchtungselement (23c) in der Nähe eines zweiten Winkels (47c) des im Wesentlichen rechtwinkligen Rahmens (41), der einem ersten Winkel (47a) gegenüberliegt, welcher ein Schnittpunkt der ersten und zweiten Seite (46a-b) ist, so dass ein Abstand zwischen dem dritten Beleuchtungselement (23c) und dem zweiten Winkel (47c) kürzer ist als ein Abstand zwischen dem dritten Beleuchtungselement (23c) und jeweiligen Mittelpunkten der Seiten (46c, 46d), die auf beiden Schenkeln des zweiten Winkels (47c) liegen, angeordnet ist,
das Einführteil (3) drei optische Führungen (33), die dem ersten, dem zweiten und dem dritten Beleuchtungselement (23a-c) entsprechen, oder mindestens eine Stromleitung zur Versorgung des ersten, des zweiten und des dritten Beleuchtungselements mit elektrischer Energie, und drei in ihm eingesetzte Einbauteile (24-26) aufweist;
distale Endteile der drei Einbauteile im Wechsel mit den drei Beleuchtungselementen (23a-c) an der distalen Endfläche des distalen Endteils (10) angeordnet sind;
eines der drei Einbauteile ein Wasserrohr (25) ist;
eine Düse (25) des distalen Endteils des Wasserrohrs (25b), das in der Nähe der ersten Seite (46a) angeordnete erste Beleuchtungselement (23a) und das Beobachtungsfenster (22) dazwischen in einer im Wesentlichen geraden Linie (P-P) an der distalen Endfläche des distalen Endteils (10) angeordnet sind; und
das Beobachtungsfenster (22) bezüglich der Mitte einer Oberfläche der distalen Endfläche (10) an einer exzentrischen Position angeordnet ist;
**dadurch gekennzeichnet, dass** ein Abstand (L2) zwischen einem Beleuchtungselement (23b), welches an der distalen Endfläche (10) an einer gegenüberliegenden Seite zur exzentrischen Position bezüglich der Mitte der Oberfläche der distalen Endfläche (10) angeordnet ist, und einer Mitte des Beobachtungsfensters (22) größer als ein Abstand (L2) zwischen einem der beiden anderen Beleuchtungselemente (23b) und der Mitte des Beobachtungsfensters (22) ist, wobei das eine Beleuchtungselement (23a-c) nicht das Beleuchtungselement (23a) ist, das in im Wesentlichen gerader Linie mit der Düse (25) angeordnet ist.

2. Endoskop nach Anspruch 1, wobei das Bildgebungselement (11) eine äußere Umfangsform aufweist, die mit zwei Seiten versehen ist, die im Wesentlichen parallel zu entsprechenden zwei gegenüberliegenden Seiten der Seiten des im Wesentlichen rechtwinkligen Rahmens (41) des Bildgebungsbereichs verlaufen.

3. Endoskop nach Anspruch 1, das des Weiteren einen Videoprozessor (6) und einen Monitor (7) aufweist, wobei der Prozessor so konfiguriert ist, dass er ein Bild verarbeitet, welches durch das Bildgebungselement (11) so erstellt wird, dass das Bild zwei Seiten aufweist, die im Wesentlichen parallel zu entsprechenden zwei gegenüberliegenden Seiten der Seiten des im Wesentlichen rechtwinkligen Rahmens (41) des Bildgebungsbereichs verlaufen, und es auf einem vorgegebenen Monitor (7) anzeigt.

4. Endoskop nach Anspruch 2, das des Weiteren einen Videoprozessor (6) und einen Monitor (7) aufweist, wobei der Prozessor so konfiguriert ist, dass er ein Bild verarbeitet, welches durch das Bildgebungselement (11) so erstellt wird, dass das Bild zwei Seiten aufweist, die im Wesentlichen parallel zu entsprechenden zwei gegenüberliegenden Seiten des äußeren Umfangsrahmens des Bildgebungselements (11) verlaufen, und es auf einem vorgegebenen Monitor (7) anzeigt.

## Revendications

1. Endoscope (1) qui comprend un élément d'imagerie (11) présentant une région d'imagerie au niveau d'une partie d'insertion (3) de celui-ci, comprenant :
une fenêtre d'observation (22) prévue au niveau d'une partie d'extrémité distale (10) de la partie d'insertion (3) pour introduire une lumière allant d'un objet à l'élément d'imagerie (11) ; et
des premier, deuxième et troisième éléments d'éclairage (23a à c) prévus autour de la fenêtre d'observation (22) sur une face d'extrémité distale de la partie d'extrémité distale (10) pour éclairer l'objet,
dans lequel :
les côtés et les angles de l'élément d'imagerie (11) et la région d'imagerie de l'élément d'imagerie (11) correspondent aux quatre côtés et aux quatre angles d'un cadre sensiblement rectangulaire (41) ;
les premier et deuxième éléments d'éclairage (23a, 23b) sont disposés près des premier et deuxième côtés attenants (46a, 46b) du cadre sensiblement rectangulaire (41) respectivement, d'une manière telle que :
une distance allant du premier élément d'éclairage (23a) à un point central du premier côté (46a) est plus courte qu'une distance allant du premier élément d'éclairage (23a) à chacun des deux angles (47a, 47d) du cadre rectangulaire (41) placés au niveau des deux extrémités du premier côté (46a), et
une distance allant du deuxième élément d'éclairage (23b) à un point central du deuxième côté (46b) est plus courte qu'une distance allant du deuxième élément d'éclairage (23b) à chacun des deux angles (47a, 47b) du cadre rectangulaire (41) placés au niveau des deux extrémités du deuxième côté (46b) ;
le troisième élément d'éclairage (23c) est disposé près d'un deuxième angle (47c) du cadre sensiblement rectangulaire (41) qui est opposé à un premier angle (47a) qui correspond à une intersection des premier et deuxième côtés (46a, 46b) d'une manière telle qu'une distance entre le troisième élément d'éclairage (23c) et le deuxième angle (47c) est plus courte qu'une distance entre le troisième élément d'éclairage (23c) et les points centraux respectifs des côtés (46c, 46d) reposant sur les deux côtés du deuxième angle (47c) ;
la partie d'insertion (3) comprend trois guides optiques (33) correspondant au premier, au deuxième, et au troisième éléments d'éclairage (23a à c) ou au moins une ligne d'alimentation électrique pour délivrer l'énergie électrique vers les premier, deuxième et troisième éléments d'éclairage, et trois parties intégrées (24 à 26) insérées à l'intérieur ;
les parties d'extrémité distale des trois parties intégrées sont disposées alternativement avec les trois éléments d'éclairage (23a à c) sur la face d'extrémité distale de la partie d'extrémité distale (10) ;
l'une des trois parties intégrées est une conduite d'eau (25b) ;
une buse (25) de la partie d'extrémité distale de la conduite d'eau (25b), le premier élément d'éclairage (23a) disposé près du premier côté (46), et la fenêtre d'observation (22) entre ceux-ci sont disposés dans une ligne sensiblement droite (P-P) sur la face d'extrémité distale de la partie d'extrémité distale (10) ; et
la fenêtre d'observation (22) est disposée au niveau d'une position décentrée par rapport au centre d'une surface de la face d'extrémité distale (10) ;
**caractérisé en ce qu'**une distance (L2) entre un élément d'éclairage (23b) disposé sur la face d'extrémité distale (10) au niveau d'un côté opposé de la position décentrée par rapport au centre de la surface de la face d'extrémité distale (10) et un centre de la fenêtre d'observation (22) est plus grande qu'une distance (L2) entre l'un des deux autres éléments d'éclairage (23b) et le centre de la fenêtre d'observation (22), l'élément d'éclairage (23a à c) n'étant pas l'élément d'éclairage (23a) disposé dans une ligne sensiblement droite avec la buse (25).

2. Endoscope selon la revendication 1, dans lequel l'élément d'imagerie (11) présente une forme périphérique externe munie de deux côtés étant sensiblement parallèles aux deux côtés opposés respectifs parmi les côtés du cadre sensiblement rectangulaire (41) de la région d'imagerie.

3. Endoscope selon la revendication 1, comprenant en outre un processeur vidéo (6) et un moniteur (7), dans lequel le processeur est configuré pour traiter une image obtenue par l'élément d'imagerie (11) de sorte que l'image présente deux côtés sensiblement parallèles aux deux côtés opposés respectifs parmi les côtés du cadre sensiblement rectangulaire (41) de la région d'imagerie, et l'afficher sur un moniteur prédéterminé (7).

4. Endoscope selon la revendication 2, comprenant en outre un processeur vidéo (6) et un moniteur (7), dans lequel le processeur est configuré pour traiter une image obtenue par l'élément d'imagerie (11) de sorte que l'image présente deux côtés sensiblement parallèles aux deux côtés opposés respectifs du cadre périphérique externe de l'élément d'imagerie (11), et l'afficher sur un moniteur prédéterminé (7).
